# EUROPÄISCHE PATENTSCHRIFT

(11) **EP 3 423 114 B1**
(45) Veröffentlichungstag und Bekanntmachung des Hinweises auf die Patenterteilung: **12.06.2024**
(21) Anmeldenummer: 17707074.5
(22) Anmeldetag: 01.03.2017
(51) Int. Cl.: A61L 2/025, A61L 2/12, B08B 7/02

(54) **VERFAHREN UND VORRICHTUNG FÜR KLEINTEILE**
PROCESS AND APPARATUS FOR SMALL PARTS
PROCÉDÉ ET DISPOSITIF DE TRAITEMENT POUR PETITES PIÈCES

(30) Priorität: 02.03.2016 CH 2682016
(43) Veröffentlichungstag der Anmeldung: 09.01.2019
(73) Patentinhaber: castus Sterile Systems GmbH & Co. KG, 88416 Ochsenhausen (DE)
(72) Erfinder: NETZHAMMER, Eric, 4144 Arlesheim (CH)
(74) Vertreter: Wallinger Ricker Schlotter Tostmann
(86) Internationale Anmeldenummer: PCT/EP2017/054803
(87) Internationale Veröffentlichungsnummer: WO 2017/149029

(56) Entgegenhaltungen:
- US-A- 4 896 010
- US-A1- 2002 159 917

## Beschreibung

Die Erfindung betrifft ein Verfahren und eine Vorrichtung zur schonenden Behandlung von Schüttmengen von Kleinteilen in einem Behälter.

Für den Zweck dieser Beschreibung wird der Begriff "Schüttmenge" für eine Menge von Kleinteilen, die ungeordnet in einen Behandlungsbehälter gefüllt, also geschüttet, im Behälter behandelt und danach immer noch ungeordnet dem Behälter entnommen werden. Diese Art der Behandlung ist für Teile, welche im pharmazeutischen oder medizinischen Bereich eingesetzt werden, also Teile von Spritzen, Ampullen etc. üblich.

Demnach bezieht sich für den Zweck dieser Beschreibung der Begriff "Kleinteile" auf die ungeordneten Einzelteile einer Schüttmenge, insbesondere Kleinteile zur Verwendung im medizinischen oder pharmazeutischen Bereich.

In bekannten Vorrichtungen zur Behandlung von solchen Kleinteilen werden folgende qualitätsrelevante Standardprozesse durchgeführt:
- Waschen zur Abreicherung von Partikeln, zur Abreicherung von Endotoxinen, zur Abreicherung von sonstigen Substanzen(z.B. organischen Resten auf der Oberfläche) etc.
- Sterilisieren mit Dampf zur Schaffung steriler Teile
- Trocknen zur Reduzierung der Restfeuchte in den Teilen und an deren Oberfläche.

Für den Waschvorgang sind die Teile in Wasser eingetaucht und von unten wird Luft eingeblasen. Damit ergibt sich ein sogenanntes Wirbelbett, welches die kleinen Teile bewegt und in Schwebe hält. Partikel und sonstige Substanzen werden dabei ausgewaschen und durch geeignete Entleerung mit dem Wasser von den kleinen Teilen getrennt.

Die bekannten Waschprozesse sind in der Lage Partikel und sonstige Substanzen abzureichern aber oft in ungenügendem Ausmass. Vor allem schwere Partikel können schlecht entfernt werden, da die bekannten Behandlungsmaschinen nur nach oben entleert werden können. Da die Kleinteile in pharmazeutischen und medizinalen Anwendungen eingesetzt werden (z.B. in Injektionsampullen), ist vor allem die Partikelabreicherung ein entscheidendes Qualitätsmerkmal.

Das Trocknen der Kleinteile wird in den bekannten Vorrichtungen mit Vakuum und Heissluft gefahren.

Die bekannten Trocknungsprozesse sind in der Lage niedrige Restfeuchten zu produzieren, welche medizinisch aber oft nicht mehr ausreichend sind. Einerseits kann sich Restwasser in den Poren der kleinen Teile befinden, welches mit den herkömmlichen Verfahren nicht herausgebracht werden kann. Andererseits kann sich Restfeuchte auch noch auf der Oberfläche befinden. Diese Restfeuchte kann bewirken, dass z.B. bei lyophilisierten Wirkstoffen, die Haltbarkeit massiv zurückgeht. Zudem kann die Trocknungszeit zur Erreichung niedriger Restfeuchte Werte mehrere Stunden dauern.

Ein weiterer Nachteil bekannte Behandlungsvorrichtungen besteht darin, dass nach einem Behandlungszyklus und der Entleerung des Behandlungsbehälters einzelne Teile im Behälter zurückbleiben können und demzufolge mehrfach behandelt werden. Vor allem aber kann eine Vermischung von Teilen unterschiedlicher Art stattfinden, was zu Problemen bei den nachfolgenden Maschinen führen kann.

Die US 2002/159917 A1 betrifft ein Verfahren und ein System zum Reinigen, Desinfizieren oder Sterilisieren von Gegenständen, wie z. B. medizinischen oder zahnärztlichen Instrumenten oder Vorrichtungen, wobei das System eine Energiequelle, wie z. B. einen Beschaller, der angepasst und konfiguriert ist, um das Objekt mit Energie, wie z. B. Ultraschallenergie, zu beaufschlagen; einen Flüssigkeitstransporter, der angepasst und konfiguriert ist, eine Waschzusammensetzung, eine antimikrobielle Zusammensetzung, eine Spülzusammensetzung oder mehrere dieser Zusammensetzungen um, durch und/oder in das Objekt zu zirkulieren; und einen Trockner umfasst, der angepasst und konfiguriert ist, um das Objekt zu trocknen, vorzugsweise in Gegenwart eines Sterilisationsmittels, und wobei das Verfahren das Kontaktieren des Objekts mit einer Waschzusammensetzung und Energie, wie etwa Ultraschallenergie; Behandeln des Objekts mit einer antimikrobiellen Zusammensetzung; Spülen des Objekts mit einer Spülzusammensetzung; und Trocknen des Objekts, vorzugsweise in Gegenwart eines Sterilisationsmittels, umfasst.

Die US 4 896 010 A betrifft ein Desinfektionsverfahren und eine Vorrichtung, die mehrere Mikrowellenquellen verwenden, wobei Bakterien durch die Kombination von mikrowellenerhitztem Wasserdampf, der sich in der abgedichteten Kammer ansammeln kann, und dem Mikrowellenfeldeffekt, der in Synergie mit dem angesammelten Wasserdampf wirkt, abgetötet werden.

Der Erfindung liegt die Auf- gabe zugrunde, die eingangs erwähnten Nachteile des Standes der Technik zu vermeiden bzw. zu verbessern.

Erfindungsgemäss wird dies erreicht durch ein Verfahren mit den Merkmalen des Anspruchs 1 bzw. eine Vorrichtung mit den Merkmalen des Anspruchs 2.

Der Behandlungsbehälter ist ein rotierender Behälter welcher in einem Wagen oder in einem Gestell drehbar ist. (Stand der Technik). Er ist je nach Anforderung mit einer Einfüllmöglichkeit und mit einem Transfersystem gemäss dem Stand der Technik versehen.

Der Behälter ist mit einem oder mehreren Ultraschallerzeuger ausgerüstet.

Der Behälter ist mit einem Lochblech ausgestattet auf welchem die kleinen Teile liegen.

Der Behälter wird mit Wasser gefüllt, sodass Ultraschallgenerator und Teile gut eingetaucht sind. Die Reinigung der Teile erfolgt nun mit Ultraschall.

Nach erfolgter Reinigung wird der Wasserpegel abgesenkt, sodass die schweren Partikel den Behandlungsbehälter nach unten verlassen können. Anschliessend wird der Flüssigkeitspegel soweit angehoben, bis ein Überlaufen stattfindet, damit werden die leichten Partikel entfernt.

Der Behälter ist mit einem oder mehreren Mikrowellengeneratoren ausgerüstet. Die Trocknung der kleinen Teile erfolgt mit Mikrowellen.

Ultraschallreinigung und Mikrowellentrocknung können kombiniert werden oder aber auch nur je einzeln zur Anwendung kommen. Das hängt u.a. von der Materialbeschaffenheit der Teile ab.

Im Folgenden werden anhand der beiliegenden Zeichnungen bevorzugte Ausführungsbeispiele der Erfindung beschrieben. Es zeigen:
- Fig. 1: eine schematische Schnittdarstellung einer erfindungsgemässen Behandlungsvorrichtung,
- Fig. 2: eine schematische Schnittdarstellung einer weiteren Form einer erfindungsgemässen Behandlungsvorrichtung im geschlossenen Zustand,
- Fig. 3: eine schematische Schnittdarstellung der in Fig. 2 gezeigten Behandlungsvorrichtung im geöffneten Zustand.

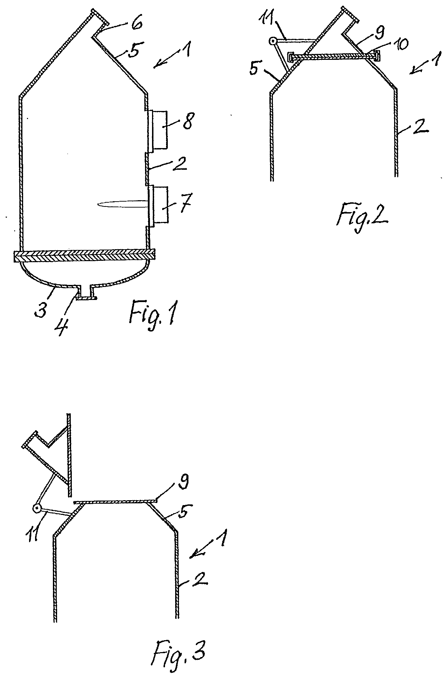

Der in Fig. 1 gezeigte Behandlungsbehälter 1 besitzt in an sich bekannter Weise eine zylindrische Seitenwand 2 mit einem angeflanschten gewölbten Boden 3. Im Bereich des Flansches ist auch in an sich bekannter weise ein Lochblech (nichtgezeigt) angeordnet. Im Boden befindet sich ein Einlaufstutzen 4 für Behandlungsmedien. Am anderen Ende des Behälters ist mit der Seitenwand ein konischer Bereich 5 verbunden, an dem sich ein Einfüll- und Entleerungsstutzen für die zu behandelnden Kleinteile 6 befindet.

An der Seitenwand ist ein Ultraschallgenerator 7 angeordnet, der den Behälterinhalt zur besseren Reinigung mit Ultraschallwellen beaufschlagt.

Ausserdem ist an der Seitenwand ein Mikrowellengenerator 8 angeordnet, der nach der Reinigungsphase zur Förderung der Trocknung den Behälterinhalt mit Mikrowellen beaufschlagt.

Bei dem in den Fig. 2 und 3 gezeigten Behandlungsbehälter ist ein Teil 9 des konischen Bereichs mittels eines Flansches 10 und einer Scharniereinrichtung 11 mit dem Behälter verbunden. Der Flansch ist in an sich bekannter Weise so ausgebildet, dass er leicht entriegelt werden kann. Dadurch kann der genannte Teil 9 des konischen Bereichs des Behälters, wie in Fig. 3 gezeigt, weggeklappt werden, um so eine ausreichend grosse Öffnung für eine manuelle Inspektion zu bilden. Durch diese Öffnung kann auch die Entleerung der behandelten Teile erfolgen, da der Behälter auf einem Wagen drehbar gelagert ist.

## Patentansprüche

1. Verfahren zur Reinigung, Sterilisierung und Trocknung von Schüttmengen von Kleinteilen in einem Behälter (1), **dadurch gekennzeichnet, dass** die Kleinteile mindestens den Schritten Ultraschallreinigung in einem Wasserbad, sowie anschliessendem Sterilisieren und Trocknen durch Beaufschlagung mit Mikrowellen unterzogen werden.

2. Vorrichtung zur schonenden Behandlung von Schüttmengen von Kleinteilen in einem Behälter, wobei die Vorrichtung
- einen Behälter (1);
- wenigstens einen Ultraschallgenerator (7) zur Beaufschlagung des Behälterinhalts mit Ultraschallwellen; und
- wenigstens einen Mikrowellengenerator (8) zur Beaufschlagung des Behälterinhalts mit Mikrowellen;
aufweist;
wobei die Vorrichtung konfiguriert ist, Schüttmengen von Kleinteilen durch Ultraschallreinigung in einem Wasserbad in dem Behälter (1) zu reinigen; **dadurch gekennzeichnet, dass** die Vorrichtung konfiguriert ist, die durch Ultraschallreinigung in dem Wasserbad behandelten Schüttmengen von Kleinteilen anschließend durch Beaufschlagung mit Mikrowellen zu trocknen.

3. Behandlungsvorrichtung nach Anspruch 2, **dadurch gekennzeichnet, dass** der Behälter (1) eine zylindrischen Seitenwand (2) und einen konischen Einfüll- und Entleerungsbereich (5) aufweist und dass ein Teil des konischen Bereichs (5) mittels einer Scharniereinrichtung (11) vom Behälter (1) wegklappbar ist.

4. Behandlungsvorrichtung nach Anspruch 3, **dadurch gekennzeichnet, dass** der wegklappbare Teil des konischen Bereichs (5) mit einem Einfüll- und Entleerungsstutzen (6) für die zu behandelnden Teile versehen ist.

5. Behandlungsvorrichtung nach einem der vorhergehenden Ansprüche, **dadurch gekennzeichnet, dass** der Behälter (1) in einem Gestell oder einem Wagen drehbar ist.

6. Behandlungsvorrichtung nach einem der vorhergehenden Ansprüche, **dadurch gekennzeichnet, dass** der Behälter (1) einen gewölbten Boden (3) besitzt, in dem ein Einfüllstutzen (4) für Behandlungsmedien angeordnet ist.

## Claims

1. A method of cleaning, sterilising and drying bulk quantities of small parts in a container (1), **characterised in that** the small parts are subjected, at least, to the steps of ultrasonic cleaning in a bath of water as well as a subsequent sterilisation and drying by application of microwaves.

2. A device for gentle treatment of bulk quantities of small parts in a container, wherein the device comprises:
- a container (1);
- at least one ultrasonic generator (7) for applying ultrasonic waves to the contents of the container; and
- at least one microwave generator (8) for applying microwaves to the contents of the container;
wherein the device is configured to clean bulk quantities of small parts by ultrasonic cleaning in a bath of water in the container (1);
**characterised in that** the device is configured to dry the bulk quantities of small parts by exposing them to microwaves after they have been treated by ultrasonic cleaning in the bath of water.

3. The treatment device as claimed in claim 2, **characterised in that** the container (1) has a cylindrical side wall (2) and a conical region (5) for filling and emptying the container (1), and **in that** a part of the conical region (5) can be pivoted away from the container (1) by means of a hinge device (11).

4. The treatment device as claimed in claim 3, **characterised in that** the part of the conical region (5) which can be pivoted away is provided with a filling and emptying nozzle (6) for the parts to be treated.

5. The treatment device as claimed in any one of the preceding claims, **characterised in that** the container (1) can be rotated in a frame or a carriage.

6. The treatment device as claimed in any one of the preceding claims, **characterised in that** the container (1) has a curved base (3) in which a filling nozzle (4) for treatment media is arranged.

## Revendications

1. Procédé de nettoyage, de stérilisation et de séchage de quantités déchargées de pièces de petite taille dans un contenant (1),
**caractérisé en ce que** les pièces de petite taille sont soumises au moins aux étapes de nettoyage par ultrasons dans un bain d'eau, puis de stérilisation et de séchage par exposition à l'action de micro-ondes.

2. Dispositif de traitement en douceur de quantités déchargées de pièces de petite taille dans un contenant, dans lequel le dispositif présente
- un contenant (1) ;
- au moins un générateur d'ultrasons (7) destiné à exposer le contenu de contenant à l'action d'ondes ultrasonores ; et
- au moins un générateur de micro-ondes (8) destiné à exposer le contenu de contenant à l'action de micro-ondes ; dans lequel le dispositif est configuré pour nettoyer des quantités déchargées de pièces de petite taille par nettoyage par ultrasons dans un bain d'eau dans le contenant (1) ;
**caractérisé en ce que** le dispositif est configuré pour sécher les quantités déchargées, traitées dans le bain d'eau par nettoyage par ultrasons, de pièces de petite taille par la suite par exposition à l'action de micro-ondes.

3. Dispositif de traitement selon la revendication 2, **caractérisé en ce que** le contenant (1) présente une paroi latérale cylindrique (2) et une zone de remplissage et de vidange conique (5), et qu'une partie de la zone conique (5) peut être écartée par rabattement du contenant (1) au moyen d'un système de charnière (11).

4. Dispositif de traitement selon la revendication 3, **caractérisé en ce que** la partie pouvant être écartée par rabattement de la zone conique (5) est pourvue d'une tubulure de remplissage et de vidange (6) pour les pièces à traiter.

5. Dispositif de traitement selon l'une quelconque des revendications précédentes, **caractérisé en ce que** le contenant (1) peut être tourné dans un châssis ou un chariot.

6. Dispositif de traitement selon l'une quelconque des revendications précédentes, **caractérisé en ce que** le contenant (1) possède un fond bombé (3), dans lequel une tubulure de remplissage (4) pour des agents de traitement est disposée.
